# EUROPEAN PATENT SPECIFICATION

(11) **EP 2 674 753 B1**
(45) Date of publication and mention of the grant of the patent: **13.05.2020**
(21) Application number: 12744859.5
(22) Date of filing: 09.02.2012
(51) Int. Cl.: G01N 30/88, B01D 15/04, B01D 15/08, B01J 41/20, B01J 20/281, G01N 30/96, C12N 15/10, B01D 15/36

(54) **METHOD FOR SEPARATING AND DETECTING A NUCLEIC ACID STRAND**
IONENAUSTAUSCH-CHROMATOGRAPHIE-VERFAHREN ZUR TRENNUNG UND DETEKTION EINES NUKLEINSÄURESTRANGS
MÉTHODE DE CHROMATOGRAPHIE PAR ÉCHANGE D'IONS POUR LA SÉPARATION ET LA DÉTECTION D'UN BRIN D'ACIDE NUCLÉIQUE

(30) Priority: 10.02.2011 JP 2011027493
(43) Date of publication of application: 18.12.2013
(73) Proprietor: Sekisui Medical Co., Ltd., Tokyo 103-0027 (JP)
(72) Inventor: YOTANI Takuya, Ibaraki 301-0852 (JP); USHIZAWA Koji, Tokyo 103-0027 (JP)
(74) Representative: Cabinet Beau de Loménie
(86) International application number: PCT/JP2012/053010
(87) International publication number: WO 2012/108516

(56) References cited:
- EP-A1- 1 437 376
- WO-A1-01/37987
- WO-A1-97/29825
- JP-A- 1 006 755
- JP-A- 5 333 015
- JP-A- 63 049 258
- JP-A- 2007 522 807
- JP-A- 2009 113 034
- US-A- 5 438 077
- MASATO ORITA ET AL.: 'Detection of polymorphisms of human DNA by gel electrophoresis as single-strand conformation polymorphisms' PROCEEDINGS OF THE NATIONAL ACADEMY OF SCIENCES vol. 86, no. 8, April 1989, pages 2766 - 2770, XP000310584

## Description

### TECHNICAL FIELD

The present disclosure relates to a filler for ion exchange chromatography used for separating and detecting a nucleic acid chain. The present invention relates to a method for separating and detecting a nucleic acid chain using the filler for ion exchange chromatography.

### BACKGROUND ART

Nucleic acids are biological macromolecules that are composed of nucleotides having a base, a sugar, and phosphoric acid, and the nucleotides are joined by phosphoester bonds. Nucleic acids are classified into deoxyribonucleic acid (DNA) and ribonucleic acid (RNA) according to a structure of a sugar.

In recent years, techniques have been developed for analyzing single nucleotide polymorphisms (SNP) which have been shown to be associated with various diseases and drug side effects; in the technique thereof, it is an important factor to accurately detect single nucleotide polymorphisms simply and in a short time.

An SSCP method (single-stranded conformation polymorphism) is known as one main method of SNP analysis (for example, Non-patent literature 1). The SSCP method is a method of detecting an SNP using a difference of conformation that is caused by a difference of the base sequence between single-stranded DNAs. However, the SSCP method needs labeling with a radioactive isotope or a fluorescent substance, resulting in a complicated process and an increase in cost of reagents. In addition, a polymorphism is separated and detected using electrophoresis, resulting in a complicated operation or long-term analysis.

In addition, an RFLP method (restriction fragment length polymorphism) is another known method of SNP analysis. The RFLP method involves, when a restriction enzyme exists recognizing a gene mutation site in a PCR amplification product, preparing primers in common sequence sites, performing amplification by holding polymorphisms in the PCR amplification product, cleaving the resultant PCR product with the restriction enzyme, and determining the presence or the absence of polymorphisms based on the length of the fragments. However, the method has problems including that the use of restriction enzyme increases analysis cost and prolongs time of the whole analysis. It also has problems including that the detection of the chain length difference by electrophoresis complicates operation and prolongs time of the whole analysis.

In the fields of biochemistry and medicine, ion exchange chromatography is commonly used as a method that enables simple, shot-time, and accurate detection for separating biological macromolecules such as nucleic acids, proteins, and polysaccharides.

Ion exchange chromatography is a method for separating a target substance using electrostatic interaction between an ion exchange group on a filler and an ion of the target substance. Ion exchange chromatography includes a type based on anion exchange and a type based on cation exchange. Anion exchange liquid chromatography enables separation of an anionic substance using a column filler having a cationic functional group, whereas cation exchange liquid chromatography enables separation of a cationic substance using a column filler having an anionic functional group.

In cases where a PCR amplification product of a nucleic acid is separated by ion exchange chromatography, the chromatography makes use of negative charge of phosphoric acid contained in a nucleic acid molecule. Therefore, the separation is performed by anion exchange liquid chromatography. The cationic functional group on a column filler for anion exchange liquid chromatography may be a weak cationic group such as a diethyl amino ethyl group or a strong cationic group such as a quaternary ammonium group. Column fillers having such a cationic functional group are already commercially available and used in various research areas.

However, ion exchange chromatography using a conventional column filler sufficiently detects neither nucleic acid chains that differ in sequence of bases nor nucleic acid chains that differ by a single base substitution.

EP 1 437 376 A1 discloses a porous sintered body of polyolefin resin particles with absorbent functional groups bonded to the surface via graft polymer chains. Independent claims are also included for: (1) polyolefin resin particles in which absorbent functional groups are bonded to the surface by graft polymer chains; and (2) the manufacture of the sintered body by sintering a particle mixture of thermoplastic resin particles of diameter = 300 µm, and crosslinked body resin particles of particle diameter = 300 µm to which absorbent functional groups can be bonded to form a resin matrix and bonding the absorbent functional groups.

US 5 438 077 A discloses an ion exchanger comprising a particle of an insoluble crosslinked polymer having alcoholic hydroxyl groups, as a base material, and a glycidyl-adduct of polyol and/or its oligomer, as a spacer, wherein ion exchange groups are attached to the spacer.

WO 01/37987 A1 discloses a method for purifying a desired substance comprising nucleic acid structure by separating substance (I) from substance (II), one of which is the desired substance, both of which have affinity for the same ligand structure, and substance (I) is smaller than substance (II). The method comprises: (i) providing substances I and II in a liquid; (ii) contacting the liquid with an adsorbent which selectively adsorbs substance I; (iii) recovering the desired substance; The adsorbent has (a) an interior part which carries a ligand structure that is capable of binding to substances I and II, and is accessible to substance I, and (b) an outer surface layer that does not adsorb substance II, and is more easily penetrated by substance I than by substance II. The use of certain anion exchangers funtionalized with a plurality of chargeable amine groups for the removal and/or purification of nucleic acid vectors. The matrices used should have (A) an increased elution ionic strength for at least one of a selection of standard proteins compared to a reference anion exchanger, or (B) have a hydroxy or amino nitrogen at a distance of 2-3 carbons from the amine groups.

WO 97/29825 A1 discloses a process for separating off a peptide or a nucleic acid by an anion exchanger (I) characterized in that a) the anion exchanger (I) exhibits ligands, which (i) contain a primary, secondary or tertiary amino group and (ii) are covalently bound to an organic polymer (matrix), b) there on a carbon atom at a distance of 2 or 3 atoms away from an amino nitrogen in the ligands is a hydroxyl group or a primary, secondary or tertiary amino group, and c) the maximum elution ionic strength in the pH range 2-14 for at least one of the proteins transferrin, ovalbumin 1, ovalbumin 2, beta-lactoglobulin 1 and beta-lactoglobulin 2 on the anion exchanger (I) is higher than the elution ionic strength required for a quaternary comparative ion exchanger. Chromatofocusing and separation on ECTEOLA-cellulose is not included. New anion exchangers which are according to structure (I) wherein R1-6 are hydrocarbon groups which can contain ether oxygen atoms, hydroxyl groups, and different types of amino groups. X is OH or alkoxy or amino. B is a ligand arm replacing a group R1-6 or a hydrogen in any of R1-6. If one of the groups R5 and R6 contains a nitrogen atom then one of the groups also contains a hydroxy or alkoxy group. ECTEOLA-cellulose and variants where both R5 and R6 are selected among methyl and ethyl are not included.

### CITATION LIST

### - Non-Patent Literature

Non-patent literature 1: Orita, M., et al.: Detection of polymorphisms of human DNA by gel electrophoresis as single-strand conformation polymorphisms. Proc. Natl. Acad. Sci. USA, 86: 2766-2770, 1989.

### - Patent Literature

EP 1 437 376 A1

US 5 438 077 A

WO 01/37987 A1

WO 97/29825 A1

### SUMMARY OF INVENTION

### - Technical Problem

The present disclosure aims to provide a filler for ion exchange chromatography which can sufficiently detect nucleic acid chains that differ in sequence of bases or nucleic acid chains that differ by a single base substitution. The present invention aims to provide a method for separating and detecting a nucleic acid chain using the filler for ion exchange chromatography.

### - Solution to Problem

The present invention relates to a method for separating and detecting a nucleic acid chain according to claim 1. One aspect of the present disclosure is a filler for ion exchange chromatography, comprising base fine particles, each particle having a strong cationic group and a weak cationic group on the surface thereof.

The present disclosure is described in detail below.

The present inventors found that use of a filler for ion exchange chromatography including base fine particles, each particle having a strong cationic group and a weak cationic group on the surface thereof, sufficiently allows the detection of nucleic acid chains that differ in sequence of bases or nucleic acid chains that differ by a single base substitution. Thereby the present disclosure was completed.

The strong cationic group herein means a cationic group that is capable of dissociating in a wide range of pH values from 1 to 14. Namely, the strong cationic group can be maintained in a dissociated (cationized) state without being affected by pH of an aqueous solution.

Specific examples of the quaternary ammonium group include a trialkylammonium group such as a trimethylammonium group, a triethylammonium group, and a dimethyl ethyl ammonium group.

A counter ion for the strong cationic group may be, for example, halide ions such as chloride ion, bromide ion, and iodide ion.

The strong cationic group may be introduced on the surface of the base fine particles in any amount. The lower limit of the amount of the strong cationic group is preferably 1 µeq/g, and the upper limit thereof is preferably 500 µeq/g, based on the dry weight of the filler. If the amount of the strong cationic group is less than 1 µeq/g, the retention ability is decreased, which may result in poor separation performance. If the amount of the strong cationic group exceeds 500 µeq/g, the retention ability is increased too high, which may lead to difficulty in eluting, and too much increase in the length of time for analysis.

The weak cationic group herein means a cationic group having a pka of 8 or higher. Namely, the weak cationic group is affected by the pH of an aqueous solution, and the dissociated state of the weak cationic group changes. Specifically, if the pH is higher than 8, the weak cationic group loses a proton, and the proportion of non-positively charged groups is therefore increased. In contrast to this, if the pH is lower than 8, the weak cationic group is protonated, and the proportion of positively charged groups is therefore increased.

According to the present invention, the weak cationic group is a tertiary amino group. The weak cationic group is introduced on the surface of the base fine particles in any amount. The lower limit of the amount of the weak cationic group is preferably 0.5 µeq/g, and the upper limit thereof is preferably 500 µeq/g. If the amount of the weak cationic group is less than 0.5 µeq/g, such too small an amount may not improve separation performance. If the amount of the weak cationic group exceeds 500 µeq/g, as in the case of the strong cationic group, the retention ability is increased too high, which may lead to difficulty in eluting, and too much an increase in the length of time for analysis.

Examples of the base fine particles include synthetic polymer fine particles obtained using a polymerizable monomer, and inorganic fine particles such as silica type particles. Hydrophobic cross-linked polymer particles made of a synthetic organic polymer are preferable.

The hydrophobic cross-linked polymer may be any of a hydrophobic cross-linked polymer obtained by copolymerization of at least one hydrophobic crosslinkable monomer and at least one reactive-functional-group-containing monomer; and a hydrophobic cross-linked polymer obtained by copolymerization of at least one hydrophobic cross-linkable monomer, at least one reactive-functional-group-containing monomer, and at least one hydrophobic non-cross-linkable monomer.

The hydrophobic crosslinkable monomer is not particularly limited as long as it has two or more vinyl groups in one monomer molecule. Examples of the hydrophobic crosslinkable monomer include di(meth)acrylic acid esters such as ethylene glycol di(meth)acrylate, polyethylene glycol di(meth)acrylate, propylene glycol di(meth)acrylate, and polypropylene glycol di(meth)acrylate; tri(meth)acrylic acid esters or tetra(meth)acrylic acid esters such as trimethylol methane tri(meth)acrylate and tetramethylolmethane tri(meth)acrylate; and aromatic compounds such as divinylbenzene, divinyltoluene, divinylxylene, and divinylnaphthalene. The (meth)acrylate herein means an acrylate or a methacrylate, and the (meth)acrylic means an acrylic or a methacrylic.

Examples of the reactive-functional-group-containing monomer include glycidyl(meth)acrylate and isocyanate ethyl(meth)acrylate.

The hydrophobic non-cross-linkable monomer is not particularly limited as long as it is a hydrophobic non-cross-linkable polymerizable organic monomer. Examples of the hydrophobic non-cross-linkable monomer include (meth)acrylic acid esters such as methyl(meth)acrylate, ethyl(meth)acrylate, butyl(meth)acrylate, and t-butyl(meth)acrylate; and styrene type monomers such as styrene and methyl styrene.

In the hydrophobic cross-linked polymer obtained by copolymerization of the hydrophobic crosslinkable monomer and the reactive-functional-group-containing monomer, the lower limit of the proportion of the segments derived from the hydrophobic crosslinkable monomer is preferably 10% by weight and more preferably 20% by weight.

In the filler for ion exchange chromatography of the present disclosure, each of the base fine particles preferably includes a polymer layer on the surface, and the polymer layer has the strong cationic group and the weak cationic group.

The strong cationic group and the weak cationic group of the polymer layer are preferably derived from independent monomers, respectively. Specifically, the filler for ion exchange chromatography of the present disclosure is preferably coated polymer particles each including a particle of the hydrophobic cross-linked polymer and a layer of a strong-cationic-group-containing hydrophilic polymer, which is copolymerized on the hydrophobic cross-linked polymer particle, and having the weak cationic group introduced on the surface of the coated polymer particles.

The strong-cationic-group-containing hydrophilic polymer is composed of a strong-cationic-group-containing hydrophilic monomer, and includes a segment derived from at least one strong-cationic-group-containing hydrophilic monomer. Namely, the strong-cationic-group-containing hydrophilic polymer may be produced, for example, by polymerization of strong-cationic-group-containing hydrophilic monomers of one kind; copolymerization of two or more kinds of strong-cationic-group-containing hydrophilic monomers; and copolymerization of a strong-cationic-group-containing hydrophilic monomer and a hydrophilic monomer without a strong cationic group.

According to the present invention, the strong-cationic-group-containing hydrophilic monomer contains a quaternary ammonium group. Specific examples of the strong-cationic-group-containing hydrophilic monomer include ethyl methacrylate triethyl ammonium chloride, ethyl methacrylate triethyl ammonium chloride, ethyl methacrylate dimethyl ethyl ammonium chloride, ethyl methacrylate dimethyl benzyl ammonium chloride, ethyl acrylate dimethyl benzyl ammonium chloride, ethyl acrylate triethyl ammonium chloride, ethyl acrylate triethyl ammonium chloride, ethyl acrylate dimethyl ethyl ammonium chloride, acrylamide ethyl trimethyl ammonium chloride, acrylamide ethyl triethyl ammonium chloride, and acrylamide ethyl dimethyl ethyl ammonium chloride.

The method for introducing the weak cationic group on the surface of the coated polymer particles may be known methods. Specific examples of the method for introducing a tertiary amino group as the weak cationic group include
a method including copolymerization of the strong-cationic-group-containing hydrophilic monomer on the surface of hydrophobic cross-linked polymer particles made of a hydrophobic cross-linked polymer that contains a segment derived from a glycidyl-group-containing monomer, and subsequent reaction of the glycidyl group with a reagent containing a tertiary amino group;
a method including copolymerization of the strong-cationic-group-containing hydrophilic monomer on the surface of hydrophobic cross-linked polymer particles made of a hydrophobic cross-linked polymer that contains a segment derived from an isocyanate-group-containing monomer, and subsequent reaction of the isocyanate group with a reagent containing a tertiary amino group;
a method of copolymerizing the strong-cationic-group-containing hydrophilic monomer with a tertiary-amino-group-containing monomer, on the surface of the hydrophobic cross-linked polymer particles;
a method of introducing a tertiary amino group on the surface of coated polymer particles with a layer of the strong-cationic-group-containing hydrophilic polymer using a tertiary-amino-group-containing silane coupling agent;
a method that includes copolymerization of the strong-cationic-group-containing hydrophilic monomer on the surface of hydrophobic cross-linked polymer particles made of a hydrophobic cross-linked polymer that contains a segment derived from a carboxy-group-containing monomer, and subsequent condensation of the carboxy group with a reagent containing a tertiary amino group using a carbodiimide; and
a method including copolymerization of the strong-cationic-group-containing hydrophilic monomer on the surface of hydrophobic cross-linked polymer particles made of a hydrophobic cross-linked polymer that contains a segment derived from an ester-bond-containing monomer, hydrolysis of the ester binding site, and subsequent condensation of a carboxy group resulting from the hydrolysis with a reagent containing a tertiary amino group using a carbodiimide.

Preferred among these are
a method including copolymerization of the strong-cationic-group-containing hydrophilic monomer on the surface of hydrophobic cross-linked polymer particles made of a hydrophobic cross-linked polymer that contains a segment derived from a glycidyl-group-containing monomer, and subsequent reaction of the glycidyl group with a reagent containing a tertiary amino group; and
a method including copolymerization of the strong-cationic-group-containing hydrophilic monomer on the surface of hydrophobic cross-linked polymer particles made of a hydrophobic cross-linked polymer that contains a segment derived from a isocyanate-group-containing monomer, and subsequent reaction of the isocyanate group with a reagent containing a tertiary amino group.

The reagent containing a tertiary amino group used for the reaction with the reactive functional group such as a glycidyl group or an isocyanate group is not particularly limited as long as it has a functional group capable of reacting with a tertiary amino group and a reactive functional group. Examples of the functional group capable of reacting with a tertiary amino group and a reactive functional group include a primary amino group and a hydroxy group. Particularly, the reagent preferably contains a primary amino group at the terminal. Specific examples of compounds as the reagent include N,N-dimethylamino methyl amine, N,N-dimethylamino ethyl amine, N,N-dimethylamino propyl amine, N,N-dimethylamino butyl amine, N,N-diethylamino ethyl amine, N,N-diethylamino propyl ethyl amine, N,N-diethylamino butyl amine, N,N-diethylamino pentyl amine, N,N-diethylamino hexyl amine, N,N-dipropylamino butyl amine, and N,N-dibutylamino propyl amine.

With respect to the positional relationship between the strong cationic group, which is a quaternary ammonium salt, and the weak cationic group, which is a tertiary amino group, the strong cationic group is preferably more distant from the base fine particle surface than the weak cationic group. In other words, the strong cationic group is located outwardly from the weak cationic group. For example, it is preferable that the weak cationic group is located within 30 Å from the surface of the base fine particle surface, and the strong cationic group is located within 300 Å of the base fine particle surface and is located outwardly from the weak cationic group.

The average particle size of the filler for ion exchange chromatography of the present disclosure is not particularly limited, and the lower limit of the average particle size is preferably 0.1 µm, and the upper limit thereof is preferably 20 µm. If the average particle size is less than 0.1 µm, the inner pressure of the filled column may be too high, causing poor separation. If the average particle size exceeds 20 µm, the dead volume in the column may be too large, causing poor separation.

The average particle size herein shows a volume average particle size, and can be measured using a particle size distribution measuring instrument (AccuSizer 780/product of Particle Sizing Systems).

Another aspect of the present invention is a method for separating and detecting a nucleic acid chain using the filler for ion exchange chromatography of the present disclosure.

In the method for separating and detecting a nucleic acid chain of the present invention, the composition of an eluent used for analysis by ion exchange chromatography may be in accordance with known conditions.

A buffer solution used for the eluent is preferably a buffer solution or organic solvent containing a known salt compound. Specific examples of the buffer solution include a Tris hydrochloric acid buffer solution, a TE buffer solution containing Tris and EDTA, a TAE buffer solution containing Tris, acetic acid, and EDTA, and a TBA buffer solution containing Tris, boric acid, and EDTA.

The pH of the eluent is not particularly limited. The lower limit of the pH is preferably 5, and the upper limit thereof is preferably 10. An eluent with a pH in the above range allows the weak cationic group to effectively work as an ion exchange group (anion exchange group). The lower limit of the pH is more preferably 6, and the upper limit thereof is more preferably 9.

Examples of the salt used for ion exchange chromatography include salts formed from an alkali metal and a halide such as sodium chloride, potassium chloride, sodium bromide, and potassium bromide; salts formed from an alkali earth metal and a halide such as calcium chloride, calcium bromide, magnesium chloride, and magnesium bromide; and inorganic acid salts such as sodium perchlorate, potassium perchlorate, sodium sulfate, potassium sulfate, ammonium sulfate, sodium nitrate, and potassium nitrate. Further, organic acid salts such as sodium acetate, potassium acetate, sodium succinate, and potassium succinate may be used.

The salt concentration of the eluent may be appropriately controlled in accordance with analysis conditions. The lower limit of the salt concentration is preferably 10 mmol/L, and the upper limit thereof is preferably 2000 mmol/L. The lower limit of the salt concentration is more preferably 100 mmol/L, and the upper limit thereof is more preferably 1500 mmol/L.

### - Advantageous Effects of Invention

According to the present disclosure, a filler for ion exchange chromatography which can sufficiently detect nucleic acid chains that differ in sequence of bases or nucleic acid chains that differ by a single base substitution is provided. Further, according to the present invention, a method for separating and detecting a nucleic acid chain using the filler for ion exchange chromatography is provided.

### BRIEF DESCRIPTION OF DRAWINGS

[Fig. 1]

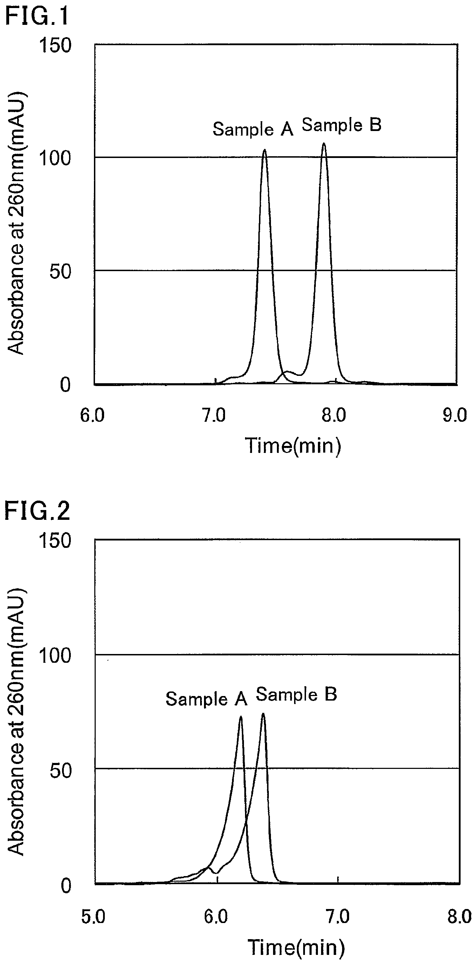

   Fig. 1 is a graph obtained by measuring oligonucleotides (samples A and B) that are different in sequence, using an anion exchange column filled with the filler produced in Example 1.
[Fig. 2]
   Fig. 2 is a graph obtained by measuring oligonucleotides (samples A and B) that are different in sequence, using an anion exchange column filled with the filler produced in Comparative Example 1.
[Fig. 3]

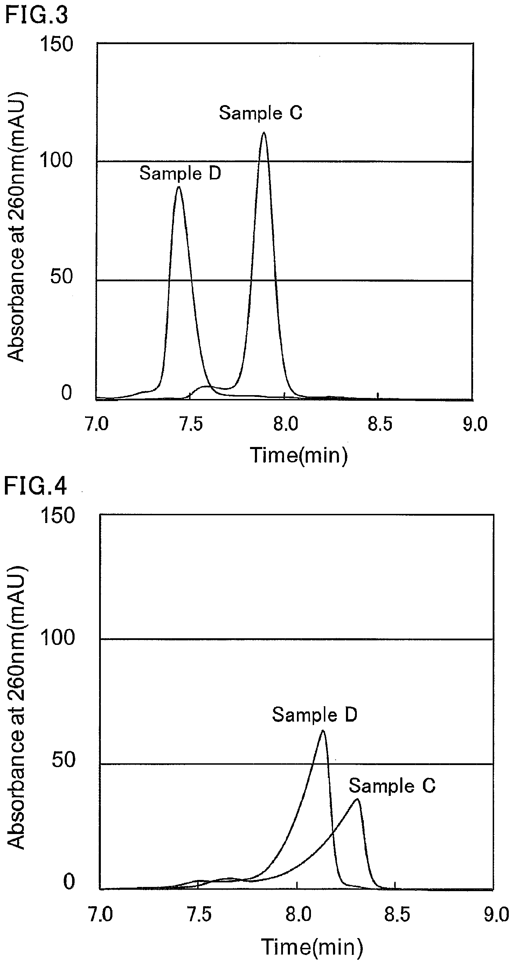

   Fig. 3 is a graph obtained by measuring oligonucleotides (samples C and D) that have a single base mismatch therebetween, using an anion exchange column filled with the filler produced in Example 1.
[Fig. 4]
   Fig. 4 is a graph obtained by measuring oligonucleotides (samples C and D) that have a single base mismatch therebetween, using an anion exchange column filled with the filler produced in Comparative Example 1.

### DESCRIPTION OF EMBODIMENTS

Hereinafter, the present invention will be described in more detail based on Examples. The present invention is not limited to those Examples.

### (Example 1)

A reactor equipped with a stirrer was charged with 2000 mL of a 3% by weight aqueous solution of polyvinyl alcohol (product of The Nippon Synthetic Chemical Industry Co., Ltd.), and to the solution was added a mixture of tetraethylene glycol dimethacrylate (product of Shin Nakamura Chemical Co., Ltd.) (200 g), triethylene glycol dimethacrylate (product of Shin Nakamura Chemical Co., Ltd.) (100 g), glycidyl methacrylate (product of Wako Pure Chemical Industries, Ltd.) (100 g), and benzoyl peroxide (product of KISHIDA CHEMICAL Co., Ltd.) (1.0 g). The contents were heated with stirring and allowed to polymerize at 80°C for 1 hour in a nitrogen atmosphere. Subsequently, ethyl methacrylate trimethyl ammonium chloride (product of Wako Pure Chemical Industries, Ltd.) (100 g) was dissolved in ion exchange water, as a strong-cationic-group-containing hydrophilic monomer. The resulting solution was added to the reactor, and the contents were similarly allowed to polymerize at 80°C for 2 hours in a nitrogen atmosphere to prepare a polymerization composition. The polymerization composition was washed with water and acetone to prepare coated polymer particles each having a layer of a quaternary-ammonium-group-containing hydrophilic polymer on the surface.

The average particle size of the coated polymer particles was measured using a particle size distribution measuring instrument (AccuSizer 780/product of Particle Sizing Systems), and was 10 µm.

Ten (10) g of the resulting coated polymer particles were dispersed in ion exchange water (100 mL) to prepare an unreacted slurry. Subsequently, N,N-dimethylamino propyl amine (product of Wako Pure Chemical Industries, Ltd.) (10 mL) was added to the slurry with stirring, and the mixture was allowed to react at 70°C for 4 hours. After the completion of the reaction, a supernatant was removed using a centrifugal separator (product of Hitachi, Ltd., "Himac CR20G"), and the remaining contents were washed with ion exchange water. After the washing, a supernatant was removed using a centrifugal separator. The washing by ion exchange water was further performed 4 times to prepare a filler for ion exchange chromatography including base fine particles, each particle having a quaternary ammonium group and a tertiary amino group on the surface thereof.

### (Comparative Example 1)

A reactor equipped with a stirrer was charged with 2000 mL of a 3% by weight aqueous solution of polyvinyl alcohol (product of The Nippon Synthetic Chemical Industry Co., Ltd.), and to the solution was added a mixture of tetraethylene glycol dimethacrylate (product of Shin Nakamura Chemical Co., Ltd.) (300 g), triethylene glycol dimethacrylate (product of Shin Nakamura Chemical Co., Ltd.) (100 g), and benzoyl peroxide (product of KISHIDA CHEMICAL Co., Ltd.) (1.0 g). The contents were heated with stirring and allowed to polymerize at 80°C for 1 hour. Subsequently, ethyl methacrylate trimethyl ammonium chloride (product of Wako Pure Chemical Industries, Ltd.) (100 g) was dissolved in ion exchange water, as a strong-cationic-group-containing hydrophilic monomer. The resulting solution was added to the same reactor, and the contents were similarly allowed to polymerize at 80°C for 2 hours in a nitrogen atmosphere to prepare a polymerization composition. The polymerization composition was washed with water and acetone to prepare coated polymer particles each having a layer of a quaternary-ammonium-group-containing hydrophilic polymer on the surface.

The average particle size of the coated polymer particles was measured using a particle size distribution measuring instrument (AccuSizer 780/product of Particle Sizing Systems), and was 10 µm.

### <Evaluation>

Each of the fillers for ion exchange chromatography produced in the example and the comparative example was filled in a stainless steel column of a liquid chromatography system (column size: 4.6 mm in inside diameter × 20 mm in length).

### <Confirmation of separation performance>

The following samples A to D were each measured in the conditions shown in Table 1 using an anion-exchange column filled with the filler for ion exchange chromatography produced in each of the example and the comparative example, and the separation performances were compared with one another.

**[Table 1]**

| | | |
|---|---|---|
| System | | LC-20A series (SHIMADZU CORPORATION) |
| Eluent | First solution | 25 mmol/L Tris hydrochloric acid buffer solution (pH 7.5) |
| | Second solution | 1 mol/L ammonium sulfate was added to 25 mmol/L Tris hydrochloric acid buffer solution (pH 7.5) |
| Elution method | | Proportion of mixed second solution lineary increased from 0% to 40% from 0 minute to 10 minutes |
| Flow rate | | 1.0 mL/min |
| Detection wavelength | | 260 nm |
| Amount of injected sample | | 2 *µ*L |

(1) Separation of oligonucleotides different in sequence Sample: Oligonucleotide 20 mer (product of Operon Biotechnologies K.K.)
   Sample A ... 5'-AACTTGAGTTCGGCGATCAC-3'
   Sample B ... 5'-CCAGCATCGATCATATTGGG-3'
      The samples A and B are different only in sequence and each include bases of five adenines, five thymines, five guanines, and five cytosines. The base sequence was randomly determined using random numbers.
(2) Separation of oligonucleotides different by single base substitution Sample: Oligonucleotide 20 mer (product of Operon Biotechnologies K.K.)
   Sample C ... 5'-CCAGCATCGATCATATTGGG-3'
   Sample D ... 5'-CCAGCATCGATCATATTGCG-3'

Figs. 1 and 2 are each a graph obtained by measuring oligonucleotides (samples A and B) different in sequence, using an anion exchange column filled with the filler produced in each of Example 1 and Comparative Example 1.

A comparison of Figs. 1 and 2 shows that use of a column filled with the filler of Example 1 including base fine particles, each particle having both a strong cationic group and a weak cationic group on the surface thereof, enables favorable separation of oligonucleotides different in sequence from each other. On the other hand, use of a column filled with the filler in Comparative Example 1 results in insufficient separation.

Figs. 3 and 4 are each a graph obtained by measuring oligonucleotides (samples C and D) having a single base mismatch therebetween, using an anion exchange column filled with the filler produced in each of Example 1 and Comparative Example 1.

A comparison of Figs. 3 and 4 shows that use of a column filled with the filler of Example 1 including base fine particles, each particle having both a strong cationic group and a weak cationic group on the surface thereof, enables favorable separation of oligonucleotides having a single base mismatch therebetween.

On the other hand, use of a column filled with the filler in Comparative Example 1 results in insufficient separation.

### INDUSTRIAL APPLICABILITY

According to the present disclosure, a filler for ion exchange chromatography which can sufficiently detect nucleic acid chains that differ in sequence of bases or nucleic acid chains that differ by a single base substitution is provided. Further, according to the present invention, a method for separating and detecting a nucleic acid chain using the filler for ion exchange chromatography is provided.

## Claims

1. A method for separating and detecting a nucleic acid chain using ion-exchange chromatography, comprising:
- providing nucleic acid chains that differ in sequence of bases or nucleic acid chains that differ by a single base substitution;
- separating and detecting nucleic acid chains with anion exchange column filled with a filler for ion exchange chromatography, comprising base fine particles, each particle having a strong cationic group and a weak cationic group on the surface thereof,
wherein the strong cationic group is a quaternary ammonium group and the weak cationic group is a tertiary amino group, and
wherein the base fine particles are hydrophobic cross-linked polymer particles made of a synthetic organic polymer.

2. The method for separating and detecting a nucleic acid chain according to claim 1,
wherein each of the base fine particles comprises a polymer layer on the surface, and the polymer layer has the strong cationic group and the weak cationic group.

## Patentansprüche

1. Verfahren zum Trennen und Detektieren einer Nukleinsäurekette unter Verwendung von lonenaustauschchromatographie, umfassend:
- Bereitstellen von Nukleinsäureketten, die sich hinsichtlich der Reihenfolge von Basen unterscheiden oder von Nukleinsäureketten, die sich durch eine Substituierung einer einzigen Base unterscheiden,
- Trennen und Detektieren von Nukleinsäureketten mit einer Anionenaustauschersäule, die für die lonenaustauschchromatographie mit Füllmaterial befüllt ist, das feine Grundpartikel umfasst, wobei jedes Partikel auf seiner Oberfläche eine stark kationische Gruppe und eine schwach kationische Gruppe aufweist,
wobei die stark kationische Gruppe eine quaternäre Ammoniumgruppe ist und die schwach kationische Gruppe eine tertiäre Aminogruppe ist und
wobei die feinen Grundpartikel hydrophobe vernetzte Polymerpartikel aus einem synthetischen organischen Polymer sind.

2. Verfahren zum Trennen und Detektieren einer Nukleinsäurekette nach Anspruch 1,
wobei jedes der feinen Grundpartikel eine Polymerschicht auf der Oberfläche umfasst und die Polymerschicht die stark kationische Gruppe und die schwach kationische Gruppe aufweist.

## Revendications

1. Procédé de séparation et détection d'une chaîne d'acide nucléique utilisant une chromatographie d'échange d'ions, comprenant :
- la fourniture de chaînes d'acides nucléiques qui diffèrent en séquence de bases ou de chaînes d'acides nucléiques qui diffèrent par une seule substitution de base ;
- la séparation et détection de chaînes d'acides nucléiques avec une colonne d'échange d'anions remplie avec une charge pour chromatographie d'échange d'ions, comprenant de fines particules de base, chaque particule présentant un groupe cationique fort et un groupe cationique faible sur sa surface,
dans lequel le groupe cationique fort est un groupe ammonium quaternaire et le groupe cationique faible est un groupe amino tertiaire, et
dans lequel les fines particules de base sont des particules polymères réticulées hydrophobes constituées d'un polymère organique synthétique.

2. Procédé de séparation et détection d'une chaîne d'acide nucléique selon la revendication 1,
dans lequel chacune des fines particules de base comprend une couche polymère sur la surface, et la couche polymère présente le groupe cationique fort et le groupe cationique faible.


### SEQUENCE LISTING

<110> SEKISUI MEDICAL CO., LTD.
<120> FILLER FOR ION EXCHANGE CHROMATOGRAPHY AND METHOD FOR SEPARATING AND
   DETECTING NUCLEIC ACID STRAND
<130> S-1341
<150> JP 2011-027493
   <151> 2011-02-10
<160> 4
<210> 1
   <211> 20
   <212> DNA
   <213> Artificial Sequence
<400> 1
   aacttgagttcggcgatcac 20
<210> 2
   <211> 20
   <212> DNA
   <213> Artificial Sequence
<400> 2
   ccagcatcgatcatattggg 20
<210> 3
   <211> 20
   <212> DNA
   <213> Artificial Sequence
<400> 3
   ccagcatcgatcatattggg 20
<210> 4
   <211> 20
   <212> DNA
   <213> Artificial Sequence
<400> 4
   ccagcatcgatcatattgcg 20